# FASCICULE DE BREVET EUROPEEN

(11) **EP 1 749 495 B1**
(45) Date de publication et mention de la délivrance du brevet: **17.10.2012**
(21) Numéro de dépôt: 06300856.9
(22) Date de dépôt: 03.08.2006
(51) Int. Cl.: A61B 18/20

(54) **Pièce à main pour appareils de traitement par émission de flashs lumineux**
Handstück für ein Behandlungsgerät mit Lichtblitzen
Handpiece for a treatment apparatus using flashes of light

(30) Priorité: 05.08.2005 FR 0552451
(43) Date de publication de la demande: 07.02.2007
(73) Titulaire: Eurofeedback, 91017 Evry Cedex (FR)
(72) Inventeur: Safraoui, Georges, 91140, Villejust (FR)
(74) Mandataire: Tanty, François

(56) Documents cités:
- EP-A- 0 775 504
- WO-A-01/87408
- AU-A- 1 851 583
- US-A- 5 626 631
- US-A1- 2003 065 314

## Description

La présente invention concerne les pièces à main pour appareils de traitement par émission de flashs lumineux, destinés par exemple à l'épilation, au photorajeunissement et/ou au traitement de défauts vasculaires ou cutanés.

La demande FR 2 864 440 décrit une pièce à main comportant une tête optique comportant au moins une lampe flash, un réflecteur en céramique et un guide optique de forme parallélépipédique.

EP 0 775 504 divulgue une pièce à main pour appareil de traitement par émission de flashs lumineux comportant une lampe flash et un guide optique en matériau transparent, ce guide optique présentant une face d'entrée de la lumière, une face de sortie d'étendue plus faible que la face d'entrée, et une surface latérale entre les faces d'entrée et de sortie, la surface latérale étant agencée pour réfléchir la lumière vers la face de sortie.

US 5 626 631 enseigne d'utiliser dans une pièce à main comportant une lampe flash un guide optique ayant un profil trapézoïdal avec une section de sortie plus faible que la section d'entrée.

Selon EP 0 775 504 et US 5 626 631, les faces latérales du guide optique font un angle constant avec l'axe du guide optique.

AU-A-18515/83 divulgue un appareil d'irradiation de tumeurs comportant une lampe incandescente et un réflecteur permettent de concenteur la lumière vers la sortie. La surface du réflecteur est lisse et la réflexion s'effectue de manière non diffuse.

La publication WO 01/87408 divulgue une pièce à main comportant un guide optique présentant un profil elliptique tronqué au niveau de la face de sortie et traversé par une cavité recevant la lampe flash et un réflecteur associé. La surface d'entrée de la lumière dans le guide est définie par la surface de la cavité. Un tel guide ne comporte pas, à proprement parler, de surface latérale s'étendant entre une face d'entrée de la lumière et une face de sortie, la lumière gagnant par l'intérieur du guide la face de sortie. Un tel guide de profil elliptique et évidé est de construction relativement délicate et la maintenance est rendue plus difficile.

Il existe un besoin pour améliorer encore le rendement des pièces à main, c'est-à-dire la densité surfacique d'énergie susceptible d'être obtenue en sortie du guide optique pour un envoi d'énergie électrique donné à la pièce à main.

L'invention vise à y répondre et a pour objet une pièce à main pour appareil de traitement par émission de flashs lumineux selon la revendication 1.

Grâce à l'invention, une énergie électrique plus faible peut être envoyée dans la lampe pour un résultat équivalent, ce qui permet d'augmenter la durée de vie de la lampe. Pour une même énergie électrique, l'invention permet d'avoir une densité surfacique d'énergie plus grande, ce qui accroît les possibilités de traitement et/ou d'avoir une étendue plus importante pour la face de sortie, ce qui permet un traitement plus rapide.

La face d'entrée peut être au moins partiellement plane, et mieux sensiblement entièrement plane, avec un éventuel chanfrein périphérique.

Dans l'invention, la lampe flash peut être extérieure au guide optique, ce qui facilite sa réalisation et la maintenance.

La face de sortie peut être au moins partiellement plane, et mieux sensiblement entièrement plane.

La portion qui fait, avec l'axe longitudinal du guide optique, un angle qui augmente en se rapprochant de la face de sortie, correspond de préférence à la majorité de la hauteur de la face et de préférence encore à la totalité de sa hauteur. L'angle peut augmenter de façon discontinue, la face précitée comportant par exemple plusieurs facettes.

La surface latérale du guide optique peut ainsi présenter au moins une face comprenant au moins une première et une seconde facettes, planes, la seconde facette étant plus proche de la face de sortie que la première, et faisant un angle avec l'axe longitudinal du guide optique supérieur à l'angle que fait la première facette avec celui-ci. La face peut présenter au moins une troisième facette, plane, adjacente à la face d'entrée et faisant avec l'axe longitudinal du guide optique un angle inférieur aux angles que font les première et deuxième facettes.

La réalisation des facettes peut se faire relativement facilement, par usinage.

Dans un exemple de mise en oeuvre de l'invention, la surface latérale présente deux faces principales, planes et parallèles, opposées, et deux faces latérales opposées non planes, convergeant généralement en direction de la face de sortie. Ces faces latérales peuvent comporter les facettes précitées.

Les faces d'entrée et de sortie peuvent présenter une forme rectangulaire, la largeur de la face de sortie étant par exemple supérieure à 10 mm, notamment comprise entre 11 et 20 mm, par exemple voisine de 15 mm.

Le guide optique étant réalisé en saphir ou en verre optique, il a de préférence une bonne conductivité thermique.

La face de sortie peut être chanfreinée, ou arrondie pour faciliter le déplacement du guide optique sur la peau, lors du traitement.

Le guide optique peut être couvert d'un revêtement opaque, par exemple une métallisation, sur au moins une partie de sa surface latérale, afin de réduire l'éblouissement du au flash de lumière.

La pièce à main comporte un réflecteur en céramique poreuse. pour réfléchir la lumière émise par la lampe flash vers la face d'entrée.

L'utilisation d'une céramique poreuse permet d'améliorer la densité d'énergie surfacique.

Le réflecteur présente avantageusement une densité intérieure ou égale à 3 g/cm³, et une porosité donnée par le rapport du volume des vides sur le volume global supérieure ou égale à 5 %, de préférence à 20 % ou 25 %, mieux à 30 %.

La céramique poreuse est émaillée partiellement, de préférence sur sa surface exposée directement à la lumière émise par la lampe flash.

La tête optique peut être parcourue par un liquide de refroidissement, par exemple de l'eau.

La céramique n'est émaillée que partiellement serrement, de façon à ce que le liquidé de refroidissement soit au contact d'au moins une portion non émaillée de la céramique. à porosité de la céramique permet au liquide de refroidissement de remplir les espaces entre les grains de la céramique, ce qui peut améliorer le renvoi de la lumière par le réflecteur.

La céramique peut présenter une face arrière entièrement non émaillée, cette face étant au contact du liquide de refroidissement.

L'invention pourra être mieux comprise à la lecture de la description détaillé qui va suivre, d'un exemple de mise en oeuvre non limitatif de celle-ci, et à l'examen du dessein annexé, sur lequel :
- la figure 1 représente de manière schématique, en élévation, un appareil de traitement
- la figure 2 est une vue en coupe, schématique et partielle, de la pièce à main de la figure 1,
- la figure 3 représente isolément, en perspective, le guide optique,
- la figure 4 est une vue de face du guide optique,
- la figure 5 représente isolément, en perspective, la tête optique,
- la figure 6 est une vue éclatée de la tête optique, et
- la figure 7 est une coupe transversale du réflecteur.

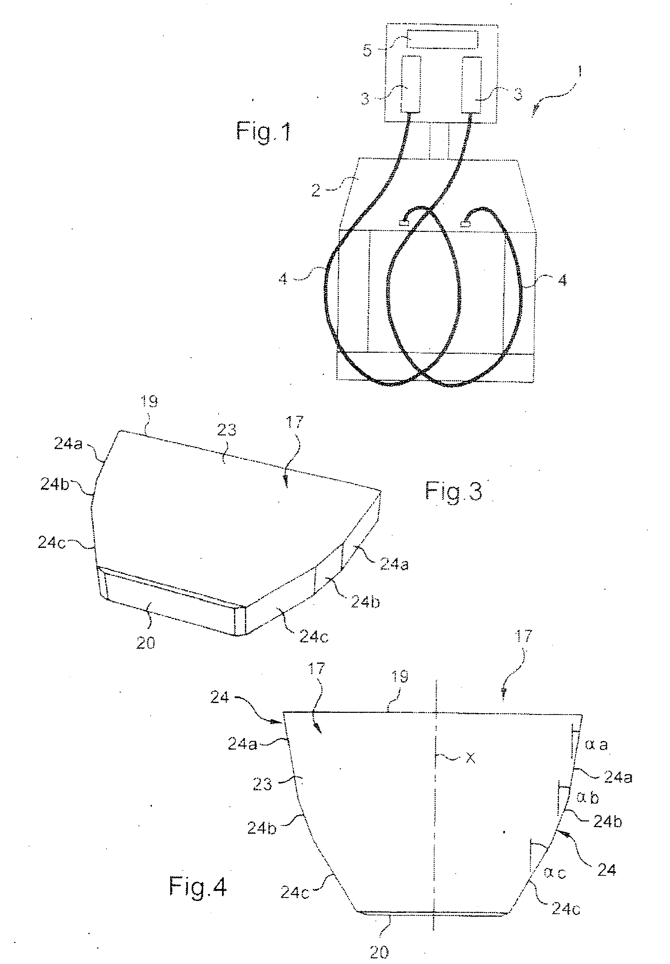

L'appareil 1 représenté à la figure 1 comporte un poste de base 2 et au moins une pièce à main 3 reliée au poste de base 2 par un câble 4. Dans l'exemple considéré, l'appareil 1 comporte deux pièces à main, permettant par exemple d'effectuer deux traitements différents.

Le poste de base 2 comporte une interface utilisateur 5 et un générateur électrique non représenté. Ce générateur est par exemple tel que décrit dans la demande FR 2 838 042.

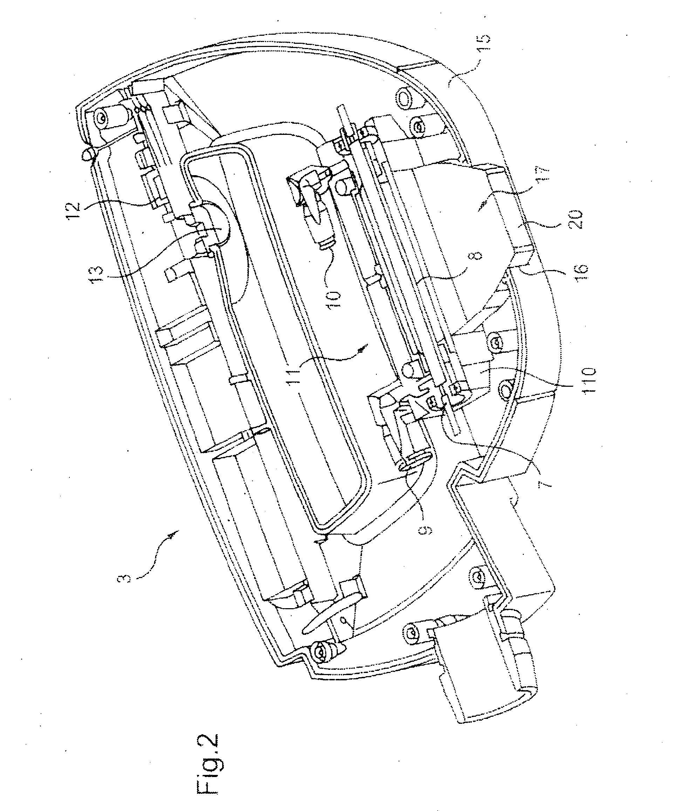

Si l'on se reporte à la figure 2, on voit que la pièce à main 3 comporte une tête optique 11 et un circuit électronique 12 comportant un interrupteur 13 permettant à l'utilisateur de déclencher l'émission d'un flash.

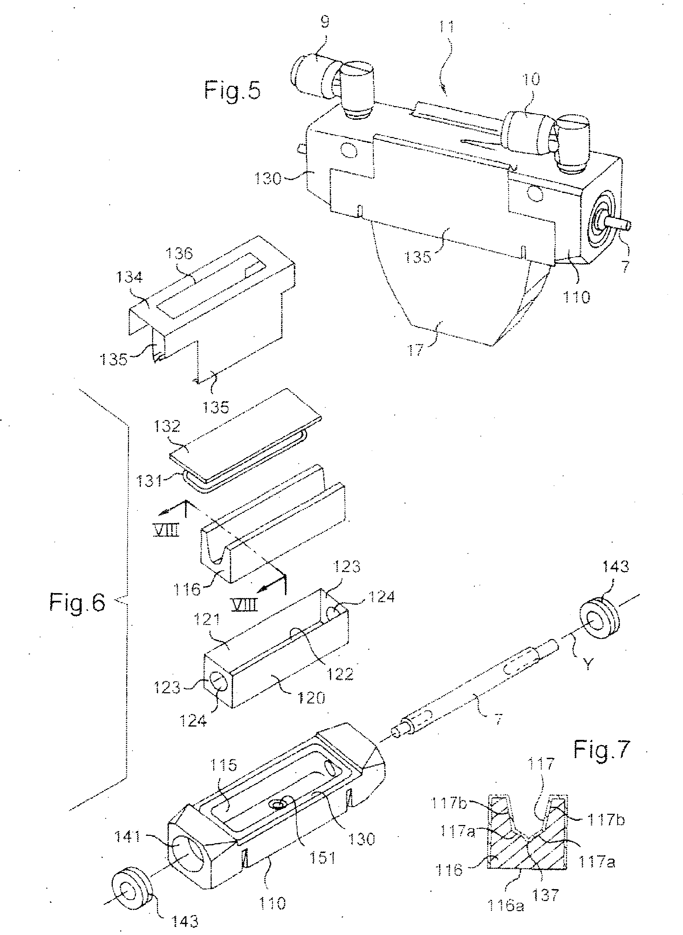

La tête optique 11 comporte, comme on peut le voir à la figure 6, au moins une lampe flash 7.

La pièce à main 3 comporte une coque 15 en matière plastique, pourvue d'une fenêtre 16 de laquelle émerge le guide optique 17.

Le guide optique 17 comporte une face d'entrée 19 recevant la lumière émise par la lampe flash 7 et une face de sortie 20 à appliquer contre la peau, avec interposition de préférence d'un gel transparent.

Dans l'exemple considéré, le guide optique 17 comporte une surface latérale formée de deux faces principales 23, planes et parallèles, et de deux faces latérales 24 composées chacune de trois facettes 24a, 24b et 24c.

Ces dernières font des angles respectifs αₐ, α_{b} et α_{c} avec l'axe longitudinal X du guide optique 17, qui augmentent lorsque l'on se rapproche de la face de sortie 20. Celle-ci est chanfreinée à sa périphérie, comme on peut le voir notamment sur la figure 3.

Le guide optique 17 est avantageusement réalisé dans un matériau qui transmet bien la chaleur, afin de favoriser le refroidissement de la zone traitée, par exemple du saphir ou un verre optique.

Les angles αₐ, α_{b} et α_{c} sont par exemple respectivement d'environ 10°, 20° et 30°. La hauteur de la facette 24a représente par exemple entre 37 % et 47 % de la hauteur totale, celle de la facette 24b entre 15 % et 25 %, et celle de la facette 24c entre 32 % et 42%.

La surface latérale peut avantageusement être métallisée afin de limiter l'éblouissement.

Le guide optique 17 permet de concentrer la lumière vers la face de sortie 20, dont la superficie est par exemple d'environ 3 à 4 cm², alors que celle de la face d'entrée est comprise entre environ 7 et 8 cm².

Ainsi, pour une énergie électrique donnée envoyée à la lampe flash 7, la densité surfacique d'énergie peut être plus importante que dans une pièce à main comportant un guide optique de forme parallélépipédique.

La largeur *l* de la face de sortie 20, hors chanfrein ou arrondi, est par exemple supérieure à 10 mm, par exemple de l'ordre de 15 mm.

Avantageusement, un gel est interposé entre la face de sortie 20 et la peau. Ce gel présente avantageusement un indice optique de réfraction relativement élevé, par exemple compris entre 1,2 et 1,6, et permet de réduire la réflexion interne de la lumière sur la face de sortie 20.

En outre, le gel est avantageusement réfrigéré avant d'être appliqué sur la peau, ce qui permet de réduire l'échauffement de la peau, notamment en cas de forte pigmentation.

On peut encore réaliser les faces principales 23 avec un angle pour les rendre convergentes vers la face de sortie 20. On peut aussi réaliser les faces latérales 4 avec un nombre différent de facettes et le cas échéant avec une évolution continue de l'angle α selon l'axe X.

On va maintenant décrire plus précisément la tête optique 11, en se référant aux figures 5 à 7.

La tête optique 11 comporte un corps 110, en matière plastique, de préférence blanche, par exemple du polytétrafluoroéthylène usiné ou du polyamide moulé, qui est traversé par le tube flash 7.

Le corps 110 comporte une cavité 115 de forme générale parallélipipédique, allongée selon un axe longitudinal Y, qui reçoit un réflecteur 116 et un bouclier thermique 120, qui s'interpose entre le réflecteur 116 et le corps 110.

Le réflecteur 116 présente une surface intérieure réfléchissante 117. Il est réalisé dans une céramique blanche poreuse, comme cela sera détaillé plus loin.

La surface réfléchissante 117 peut présenter des formes diverses sans que l'on sorte du cadre de la présente invention. Dans l'exemple considéré, le réflecteur 116 présente un fond 117a et des flancs 117b sensiblement plans.

Le bouclier 120 est réalisé, dans l'exemple considéré, par découpage et pliage d'une tôle, par exemple de l'acier inoxydable à ressort, et présente deux parois longitudinales 121, une paroi de fond 122 et deux parois d'extrémité 123, traversées chacune par une ouverture 124 permettant le passage du tube flash 7.

Le corps 110 présente, sur sa face tournée du côté du guide optique 17, une gorge 130 pour accueillir un joint d'étanchéité 131 contre lequel est pressé un filtre 132, par exemple de couleur rouge, lequel est lui-même maintenu comprimé contre le joint 131 par une pièce de maintien 134 comportant deux montants 135 encliquetés sur des reliefs prévus à cet effet sur la face du corps 110 située à l'opposé du guide optique 17.

Le filtre 132 peut être choisi en fonction de l'utilisation, par exemple de la couleur des poils à éliminer ou de la nature du traitement, notamment épilation ou photorajeunissement ou autre.

La pièce de maintien 134 comporte une ouverture 136 dont la section correspond sensiblement à celle du guide optique 17 et dans laquelle celui-ci est engagé à une extrémité.

On peut voir sur la figure 7 que le réflecteur 116 comporte une gorge longitudinale 137 dans laquelle est engagé un fil conducteur, qui fait le tour du réflecteur dans le sens de sa longueur, qui est en contact avec le bouclier 120 et qui facilite l'allumage du tube flash 7.

La cavité 115 est délimitée axialement par des régions d'extrémité qui s'étendent généralement perpendiculairement à l'axe Y et dans lesquelles débouchent des ouvertures 141 destinées au passage du tube 7. Ces ouvertures 141 débouchent à l'extérieur du corps 110 par des lamages permettant d'accueillir des bouchons 143.

Le corps 110 est traversé par un trou 150 qui débouche dans le fond de la cavité 15 et qui permet la fixation d'une vis 151. La tête de cette vis 151 vient en appui dans le fond d'un lamage et obture de manière étanche le trou 150.

La vis 151 est reliée à un conducteur électrique d'amenée d'une tension d'allumage du tube.

Un contact électrique existe entre le bouclier 120 et le conducteur d'amenée de cette tension d'allumage.

Pour refroidir le tube flash 7, une circulation d'un liquide de refroidissement, en l'espèce de l'eau déminéralisée et/ou désionisée est organisée dans la cavité 115.

Le liquide de refroidissement est amené par des canaux intérieurs communiquant avec des embouts 9 et 10 de raccordement à des tubulures d'amenée et de départ du liquide de refroidissement.

L'homme du métier pourra utilement se référer à la demande FR 2 864 440 qui décrit une tête optique dont les particularités de réalisation pourront utilement être reprises.

Le réflecteur 116 est réalisé conformément à l'un des aspects de l'invention dans une céramique poreuse de densité inférieure ou égale 3 g/cm³, par exemple de l'ordre de 2 g/cm³ et de porosité supérieure à 5 %, de préférence à 30 % environ. La céramique est émaillée sur sa surface extérieure à l'exception de sa face arrière 116a qui est au contact du liquide de refroidissement, ce qui permet ainsi à celui-ci de pénétrer entre les grains de céramique.

Le rendement du réflecteur s'en trouve augmenté.

L'invention n'est pas limitée à l'exemple qui vient d'être décrit. Par exemple, seul l'un du réflecteur et du guide optique peut être réalisé conformément à l'invention.

L'expression « comportant un » doit être comprise comme étant synonyme de « comportant au moins un », sauf si le contraire est spécifié.

## Revendications

1. Pièce à main (3) pour appareil de traitement par émission de flashs lumineux, comportant au moins une lampe flash (7) et au moins un guide optique (17) en un matériau transparent, réalisé en saphir ou en verre optique, ce guide optique présentant une face (19) d'entrée de la lumière, une face (20) de sortie, d'étendue plus faible que la face d'entrée, et une surface latérale entre les faces d'entrée et de sortie, la surface latérale étant agencée pour réfléchir la lumière vers la face de sortie de manière à ce que la densité surfacique d'énergie quittant la face de sortie soit plus élevée que celle entrant par la face d'entrée, la pièce à main comportant en outre un réflecteur (116) en céramique poreuse pour refléchir la lumière émise par la lampe flash (7) vers la face d'entrée (19) **caractérisée en ce que** la surface latérale comporte au moins une face (24) non plane, dont une portion au moins fait, avec l'axe longitudinale (X) du guide optique, un angle (α) qui augmente en se rapprochant de la face de sortie (20), et par le fait que la céramique n'est émaillée que partiellement seulement, que la pièce à main comprend une circulation d'un liquide de refroidissement et que le liquide de refroidissement vient au contact d'au moins une portion non émaillée de la céramique.

2. Pièce à main selon la revendication 1, dans laquelle la face de sortie est au moins partiellement plane.

3. Pièce à main scion la revendication 1, dans laquelle l'angle (α) augmente de façon discontinue, la face non plane (24) comportant plusieurs facettes.

4. Pièce à main selon l'une quelconque des revendications précédentes, dans laquelle la dite face non place (24) comprend au moins une première (24b) et une seconde (24c) facettes, planes, la seconde facette (24c) étant plus proche de la face de sortie (20) que la première (24), et faisant un angle (α_{c}) avec l'axe longitudinal (X) du guide optique (17) supérieur à l'angle (α_{b}) que fait la première facette (24b) avec celui-ci.

5. Pièce à main selon la revendication précédente, dans laquelle la surface comprend au moins une troisième facette (24a) adjacente à la face d'entrée (10) et faisant un angle (αₐ) avec l'axe longitudinal (X) du guide optique inférieur aux angles (α_{b}, α_{c}) que font les première (24b) et deuxième facettes (24c).

6. Pièce à main selon l'une quelconque des revendications précédentes, dans laquelle la surface latérale présente deux faces principales (23) planes et parallèles. opposées, et deux faces latérales (24) opposées non planes, convergeant genéralement en direction de la face de sortis.

7. Pièce à mam selon l'une quelconque des revendications 1 à 6, dans laquelle les faces d'entrée (19) et de sortie (20) présentent une forme rectangulaire.

8. Pièce à main selon l'une quelconque des revendications 1 à 7, la face de sortie (20) étant chanfreinée ou arrondie.

9. Pièce à main selon l'une quelconque des revendications 1 à 8, la surface latérale étant au moins partiellement recouverte d'un revêtement opaque, notamment; une métallisation, de façon à limiter l'éblouissement.

10. Pièce à main selon la revendication 1 le réflecteur présentant une densité inférieure ou égale à 3 g/cm^{3.}

11. Pièce à main selon la revendication 10, dans laquelle la céramique présente une porosité supérieure ou égale à 5 %.

12. Pièce a main selon la revendication 10, dans laquelle la porosité est supérieure ou égale à 20 %, mieux 25 % ou 30% précédentes.

13. Pièce à main selon l'un des revendications 1 à 12, dans laquelle le réflecteur présente une face arrière (116a) entièrement non émaillée et au contact du liquide de refroidissement.

## Claims

1. Handpiece (3) for a treatment apparatus using flashes of light, comprising at least one flash lamp (7) and at least one optical guide (17) of transparent material, made of sapphire or optical glass, this optical guide comprising a light inlet face (19), an outlet face (20) of (20) of smaller extent that the inlet face, and a side surface between the inlet and outlet faces, the side surface being adapted to reflect light towards the outlet face such that the surface density of energy leaving the outlet face is higher than that entering via the inlet face, the handpiece comprising also a porous ceramic reflector (116) to reflect the light emitted by the flash lamp (7) towards the inlet face (19), **characterised in that** the side surface comprises at least one non-flat face (24) of which at least one portion makes the longitudinal axis (X) of the optical guide an angle (α) which increases as it approaches the outlet face (20), and **in that** the ceramic is glazed only partially, that the handpiece comprises a circulation of a cooling liquid and that the cooling liquid is in contact with at least one unglazed portion of the ceramic.

2. Handpiece according to claim 1, wherein the outlet face is at least partially flat.

3. Handpiece according to claim 1, wherein the angle (a) increases discontinuously, the non-flat face (24) comprising several facets.

4. Handpiece according to any of the preceding claims, wherein said non-flat face (24) comprises at least a first (24b) and a second (24c) flat facet, the second facet (24c) being closer to the outlet face (20) than the first (24) and making an angle (α_{c}) with the longitudinal axis (X) of the optical guide (17) which is greater than the angle (α_{b}) made by the first facet (24b) therewith.

5. Handpiece according to the preceding claim, wherein the side surface comprises at least one third facet (24a) adjacent to the inlet face (19) and making an angle (αₐ) with the longitudinal axis (X) of the optical guide which is less than the angles (α_{b}, α_{c}) made by the first (24b) and second (24c) facets.

6. Handpiece according to any of the preceding claims, wherein the side surface has two flat, parallel and opposite main faces (23) and two opposite non-flat side faces (24) generally converging in the direction of the outlet face.

7. Handpiece according to any of claims 1 to 6, wherein the inlet face (19) and outlet face (20) have a rectangular form.

8. Handpiece according to any of claims 1 to 7, wherein the outlet face (20) is chamfered or rounded.

9. Handpiece according to any of claims 1 to 8, wherein the side surface is at least partially covered with an opaque coating, in particular a metal coating, so as to limit the dazzle.

10. Handpiece according to claim 1, wherein the reflector has a density less than or equal to 3 g/cm³.

11. Handpiece according to claim 10, wherein the ceramic has a porosity greater than or equal to 5%.

12. Handpiece according to claim 10, wherein the porosity is greater than or equal to 20%, preferably 25% or 30%.

13. Handpiece according to any of claims 1 to 12, wherein the reflector has a rear face (116a) which is fully unglazed and in contact with the cooling liquid.

## Patentansprüche

1. Handstück (3) für ein Behandlungsgerät mit Lichtblitzen, das wenigstens eine Blitzlampe (7) und wenigstens einen optischen Wellenleiter (17) aus einem transparenten Material aufweist, der aus Saphir oder optischem Glas gebildet ist, wobei dieser optische Wellenleiter eine Eintrittsfläche (19) für das Licht, eine Austrittsfläche (20) von geringerer Ausdehnung als die Eintrittsfläche und eine seitliche Oberfläche zwischen den Eintritts- und Austrittsflächen aufweist, wobei die seitliche Oberfläche so ausgebildet ist, dass sie das Licht so zu der Austrittsfläche reflektiert, dass die Flächendichte der Energie, die die Austrittsfläche verlässt, größer ist als bei Eintritt durch die Eintrittsfläche, wobei das Handstück außerdem einen Reflektor (116) aus poröser Keramik zum Reflektieren des von der Blitzlampe (7) emittierten Licht zu der Eintrittsfläche (19) aufweist, **dadurch gekennzeichnet dass** die seitliche Oberfläche wenigstens eine nicht ebene Fläche (24) aufweist, von der zumindest ein Teil mit der Längsachse (X) des optischen Wellenleiters einen Winkel (α) bildet, der bei Annäherung an die Austrittsfläche (20) zunimmt, und dadurch, dass die Keramik nur zum Teil emailliert ist, dass das Handstück einen Kreislauf für Kühlflüssigkeit aufweist, und dass die Kühlflüssigkeit zumindest mit einem nicht emaillierten Teil der Keramik in Kontakt kommt.

2. Handstück nach Anspruch 1, bei dem die Austrittsfläche zumindest zum Teil eben ist.

3. Handstück nach Anspruch 1, bei dem der Winkel (α) stetig zunimmt, wobei die nicht ebene Fläche (24) mehrere Facetten aufweist.

4. Handstück nach einem der vorstehenden Ansprüche, bei dem die genannte nicht ebene Fläche (24) wenigstens eine erste ebene Facette (24d) und eine zweite ebene Facette (25c) aufweist, die zweite Facette (24c) der Austrittsfläche (20) näher liegt als die erste (24) und mit der Längsachse (X) des optischen Wellenleiters (17) einen Winkel (α_{c}) bildet, der größer ist als der Winkel (α_{b}), den die erste Facette (24b) mit dieser Achse bildet.

5. Handstück nach dem vorstehenden Anspruch, bei dem die seitliche Oberfläche wenigstens eine dritte Facette (24a) aufweist, die sich an die Eintrittsfläche (19) anschließt und mit der Längsachse (X) des optischen Wellenleiters einen Winkel (αₐ) bildet, der kleiner ist als die Winkel (α_{b}, α_{c}), den die erste (24b) und die zweite Facette (24c) bilden.

6. Handstück nach einem der vorstehenden Ansprüche, bei dem die seitliche Oberfläche zwei ebene und parallele Hauptflächen (23) bildet, die einander gegenüberliegen, und zwei gegenüberliegende nicht ebene seitliche Flächen (24), die allgemein in Richtung auf die Austrittsfläche konvergieren.

7. Handstück nach einem der Ansprüche 1 bis 6, bei dem die Eintrittsfläche (19) und die Austrittsfläche (20) eine rechteckige Form haben.

8. Handstück nach einem der Ansprüche 1 bis 7, bei dem die Austrittsfläche (20) abgefast oder abgerundet ist,

9. Handstück nach einem der Ansprüche 1 bis 8, bei dem die seitliche Oberfläche zumindest zum Teil von einer nicht lichtdurchlässigen Abdeckung, insbesondere Metallisierung bedeckt ist, in der Weise, dass die Blendung begrenzt wird.

10. Handstück nach Anspruch 1, bei dem der Reflektor eine Dichte kleiner oder gleich 3 g/cm³ hat.

11. Handstück nach Anspruch 10, bei dem die Keramik eine Porosität größer oder gleich 5 % hat.

12. Handstück nach Anspruch 10, bei dem die Porosität größer oder gleich 20 %, besser 25 % oder 30% ist.

13. Handstück nach einem der vorstehenden Ansprüche 1 bis 12, bei dem der Reflektor eine rückwärtige Fläche (116a) hat, die in ihrer Gesamtheit nicht emailliert ist und mit der Kühlflüssigkeit in Kontakt ist.
